Europäisches Patentamt

European Patent Office

Office européen des brevets

(19)

Veröffentlichungsnummer: **0 297 355**
**A1**

## EUROPÄISCHE PATENTANMELDUNG

(21) Anmeldenummer: 88109563.2

(22) Anmeldetag: 15.06.88

(51) Int. Cl.⁴: **A61B 6/00**

(30) Priorität: 30.06.87 DE 3721590

(43) Veröffentlichungstag der Anmeldung:
04.01.89 Patentblatt 89/01

(84) Benannte Vertragsstaaten:
**BE DE FR GB**

(71) Anmelder: **Siemens Aktiengesellschaft Berlin
und München
Wittelsbacherplatz 2
D-8000 München 2(DE)**

(72) Erfinder: **Hüttenrauch, Gerd
Sudetenstrasse 14
D-8525 Uttenreuth(DE)**
Erfinder: **Jenner, Erhard
Lerchenstrasse 47
D-8521 Hessdorf(DE)**

(54) Röntgendiagnostikanlage zur wahlweisen Durchleuchtung oder Aufnahme eines Untersuchungsobjektes.

(57) Die Erfindung betrifft eine Röntgendiagnostikanlage zur wahlweisen Durchleuchtung oder Aufnahme eines Untersuchungsobjektes mit einem Röntgenbildverstärker (7) mit nachgeschalteter Fernsehkette, einem Blattfilmwechsler (13) sowie einem Röntgenstrahler (6) und einer Liege (9) für das Untersuchungsobjekt, wobei der Blattfilmwechsler (13) ein Fahrgestell (14) aufweist. Der Röntgenstrahler (6) und der Röntgenbildverstärker (7) sind gemeinsam längs der Liege (9) verschiebbar und der Blattfilmwechsler (13) ist mittels des Fahrgestells (14) im Strahlengang vor dem Röntgenbildverstärker (7) positionierbar und mit diesem derart kuppelbar, daß er Bewegungen des Röntgenbildverstärkers (7) längs der Liege (9) auf seinem Fahrgestell (14) folgt.

FIG 1

EP 0 297 355 A1

# Röntgendiagnostikanlage zur wahlweisen Durchleuchtung oder Aufnahme eines Untersuchungsobjektes

Die Erfindung betrifft eine Röntgendiagnostikanlage zur wahlweisen Durchleuchtung oder Aufnahme eines Untersuchungsobjektes mit einem Röntgenbildverstärker mit nachgeschalteter Fernsehkette, einem Blattfilmwechsler sowie einem Röntgenstrahler und einer Liege für das Untersuchungsobjekt, wobei der Blattfilmwechsler ein Fahrgestell aufweist.

Derartige Röntgendiagnostikanlagen bieten die Möglichkeit, von einem im Durchleuchtungsbetrieb erkennbaren diagnostisch relevanten Objekt eine Direktaufnahme anfertigen zu können.

Eine Röntgendiagnostikanlage der eingangs genannten Art ist aus der Druckschrift "Puck" der Fa. Siemens-Elema AB, Schweden, Druckzeichen: WS 4788, bekannt. Dabei ist der Röntgenbildverstärker oberhalb der Liege an einem Deckenstativ angeordnet, während sich der Röntgenstrahler in einem Sockel der Liege befindet, die eine längsverschiebbare Lagerungsplatte für das Untersuchungsobjekt besitzt. Der Blattfilmwechsler ist mittels seines Fahrgestells unmittelbar neben dem Sockel der Liege derart positionierbar, daß ein zunächst mittels des Röntgenstrahlers und des Röntgenbildverstärkers durchleuchteter Bereich des Untersuchungsobjektes durch Längsverschieben der Lagerungsplatte anschließend zur Anfertigung einer Aufnahme vor dem Blattfilmwechsler positioniert werden kann. Zur Anfertigung der Aufnahme ist ein zweiter, an einem Deckenstativ oberhalb des Blattfilmwechslers angeordneter Röntgenstrahler erforderlich. Abgesehen davon, daß zwei Röntgenstrahler erforderlich sind, wird es in der Praxis als nachteilig empfunden, daß eine Verschiebung des Untersuchungsobjektes erforderlich ist, um vom Durchleuchtungsbetrieb zum Aufnahmebetrieb überzugehen. Dies gilt besonders im Falle von angiographischen Untersuchungen, bei denen das Untersuchungsobjekt häufig katheterisiert oder an Kontrastmittel-Injektoren angeschlossen ist, so daß eine Verschiebung des Untersuchungsobjektes mit Problemen verbunden ist.

In der obengenannten Druckschrift ist außerdem ein Röntgendiagnostikgerät beschrieben, bei dem nur ein einziger Röntgenstrahler zur Durchleuchtung und Aufnahme erforderlich ist, wobei der Röntgenbildverstärker und der Blattfilmwechsler in Längsrichtung der Liege nebeneinander in deren Sockel unterhalb der Lagerungsplatte angeordnet sind und der oberhalb der Lagerungsplatte angebrachte Röntgenstrahler gemeinsam mit dieser in deren Längsrichtung verschiebbar ist. Um von einem zunächst durchleuchteten Bereich des Untersuchungsobjektes eine Aufnahme anzufertigen, wird der Röntgenstrahler gemeinsam mit der Lagerungsplatte und dem darauf liegenden Untersuchungsobjekt ausgehend von einer Stellung, in der er dem Röntgenbildverstärker gegenüberliegend angeordnet ist, in eine Stellung verschoben, in der er dem Blattfilmwechsler gegenüberliegt. Nachteilig an dieser Röntgendiagnostikanlage ist, daß der Blattfilmwechsler fester Bestandteil derselben ist und somit nicht beliebig verwendbar ist. Außerdem ist es aus den oben erwähnten Gründen von Nachteil, daß das Untersuchungsobjekt verschoben werden muß, um vom Durchleuchtungs- zum Aufnahmebetrieb übergehen zu können.

Weiter ist in der oben erwähnten Druckschrift eine Röntgendiagnostikanlage beschrieben, bei der der Röntgenbildverstärker und der Röntgenstrahler an entgegengesetzten Enden eines allseitig - schwenkbaren und höhenverstellbaren bogenförmigen Trägers angebracht sind. Dabei ist der Blattfilmwechsler mit dem Röntgenbildverstärker zu einer Einheit verbunden, die um eine Achse derart - schwenkbar ist, daß wahlweise der Röntgenbildverstärker oder der Blattfilmwechsler mit dem Röntgenstrahler zusammenwirken kann. Diese Röntgendiagnostikanlage gestattet es, auf einfache Weise vom Durchleuchtungs- zum Aufnahmebetrieb überzugehen, nämlich durch geeignetes Schwenken der aus Röntgenbildverstärker und Blattfilmwechsler gebildeten Einheit, ohne daß dazu Verschiebungen des Untersuchungsobjektes erforderlich sind. Allerdings ist nachteilig, daß der Bogen in Längsrichtung der Liege ortsfest angebracht ist, so daß während bestimmter Untersuchungen nach wie vor eine Längsverschiebung der Lagerungsplatte mit dem daraufliegenden Untersuchungsobjekt erforderlich ist, z.B. im Falle angiographischer Untersuchungen, bei denen der Verlauf eines in das Untersuchungsobjekt injizierten Kontrastmittels in längs der Liege aufeinanderfolgenden Bereichen des Untersuchungsobjektes beobachtet bzw. aufgenommen werden soll. Außerdem ist der Blattfilmwechsler fester Bestandteil der Röntgendiagnostikanlage und kann nicht unabhängig von dieser Verwendung finden.

Der Erfindung liegt die Aufgabe zugrunde, eine Röntgendiagnostikanlage der eingangs genannten Art so auszubilden, daß weder beim Übergang vom Durchleuchtungs- zum Aufnahmebetrieb noch bei der Untersuchung von längs der Liege aufeinanderfolgenden Bereichen des Untersuchungsobjektes Verschiebungen des Untersuchungsobjektes erforderlich sind, daß für die Durchleuchtung und die Aufnahme eines Untersuchungsobjektes nur ein einziger Röntgenstrahler erforderlich ist, daß die Ausrichtung zwischen Röntgenbildverstärker und Röntgenstrahler auch im Aufnahmebetrieb beibehalten werden kann und daß der Blattfilmwechsler separat verwendbar ist.

Nach der Erfindung wird diese Aufgabe dadurch gelöst, daß der Röntgenstrahler und der Röntgenbildverstärker gemeinsam längs der Liege verschiebbar sind und daß der Blattfilmwechsler mittels des Fahrgestells im Strahlengang vor dem Röntgenbildverstärker positionierbar und mit diesem derart kuppelbar ist, daß er Bewegungen des Röntgenbildverstärkers längs der Liege auf seinem Fahrgestell folgt. Es wird deutlich, daß somit zur Durchleuchtung und Aufnahme nur ein einziger Röntgenstrahler erforderlich ist und die Ausrichtung des Röntgenstrahlers zum Röntgenbildverstärker bestehen bleibt, da der Blattfilmwechsler mittels seines Fahrgestells im Strahlengang vor dem Röntgenbildverstärker positionierbar ist. Außerdem sind Verschiebungen des Untersuchungsobjektes beim Übergang vom Durchleuchtungs-zum Aufnahmebetrieb offensichtlich nicht erforderlich. Da der Röntgenstrahler und der Röntgenbildverstärker gemeinsam längs der Liege verschiebbar sind und der Blattfilmwechsler mit dem Röntgenbildverstärker derart kuppelbar ist, daß er dessen Bewegungen längs der Liege auf seinem Fahrgestell folgt, kann das Untersuchungsobjekt auch dann in Ruhe bleiben, wenn während der Untersuchung Verschiebungen des Röntgenstrahlers und des Röntgenbildverstärkers sowie des gegebenenfalls mit letzterem gekuppelten Blattfilmwechslers längs der Liege erforderlich sein sollten. Der Blattfilmwechsler, der sich von einem handelsüblichen Blattfilmwechsler mit Fahrgestell nur durch eine Einrichtung unterscheidet, mittels derer er mit dem Röntgenbildverstärker kuppelbar ist, kann auch unabhängig von der Röntgendiagnostikanlage Verwendung finden.

Falls bei der Röntgendiagnostikanlage in an sich bekannter Weise der Röntgenbildverstärker und der Röntgenstrahler an entgegengesetzten Enden eines allseitig schwenkbaren und höhenverstellbaren bogenförmigen Trägers angebracht und Mittel zum motorischen Bewegen des Trägers und eine Steuerungseinrichtung für die Bewegungen des Trägers vorgesehen sind, ist nach einer Variante der Erfindung vorgesehen, daß der Röntgenbildverstärker in eine Kuppelposition zum Ankuppeln des Blattfilmwechslers verfahrbar und die Steuerungseinrichtung derart ausgebildet ist, daß in der Kuppelposition bei vor dem Röntgenbildverstärker befindlichem Blattfilmwechsler sämtliche Bewegungen des Trägers bis zum erfolgten Ankuppeln des Blattfilmwechslers blockiert sind und nach erfolgtem Ankuppeln des Blattfilmwechslers Bewegungen des Trägers nur längs der Liege möglich sind. Durch diese Maßnahme wird sichergestellt, daß sich der Blattfilmwechsler problemlos an den Röntgenbildverstärker ankuppeln läßt. Außerdem ist es so ausgeschlossen, daß durch unbeabsichtigte Bewegungen des Röntgenbildverstärkers bei vor diesem positionierten Blattfilmwechsler Beschädigungen des Röntgenbildverstärkers oder des Blattfilmwechslers auftreten. Aus dem gleichen Grunde sind nach erfolgtem Ankuppeln des Blattfilm wechslers Bewegungen nur längs der Liege möglich. Um die Bewegungen in der jeweils erforderlichen Weise blockieren zu können, sieht eine Ausführungsform der Erfindung vor, daß mit der Steuerungseinrichtung zusammenwirkende erste Mittel, mittels derer die Anwesenheit des Blattfilmwechslers vor dem Röntgenbildverstärker feststellbar ist, und zweite Mittel vorgesehen sind, mit deren Hilfe das erfolgte Ankuppeln des Blattfilmwechslers an den Röntgenbildverstärker feststellbar ist.

In der Regel wird sich der Röntgenbildverstärker in der Kuppelposition unterhalb der Liege befinden.

Das Fahrgestell des Blattfilmwechslers weist nach einer Ausführungsform der Erfindung einen den Röntgenbildverstärker in Kuppelposition umgreifenden U-förmigen Rahmen mit zwei horizontalen und einem vertikalen Schenkel auf, wobei der eine horizontale Schenkel den Blattfilmwechsler trägt und der andere horizontale Schenkel mit Rädern versehen ist. Durch diese Ausbildung des Fahrgestells ist es möglich, den Blattfilmwechsler problemlos an den in seiner Kuppelposition befindlichen Röntgenbildverstärker heranzufahren und mit diesem zu kuppeln.

Ein Ausführungsbeispiel der Erfindung ist in den beigefügten Zeichnungen dargestellt. Es zeigen:

Fig. 1 eine erfindungsgemäße Röntgendiagnostikanlage in perspektivischer Darstellung, und

Fig. 2 und 3 Einzelheiten der erfindungsgemäßen Röntgendiagnostikanlage in schematischer Darstellung.

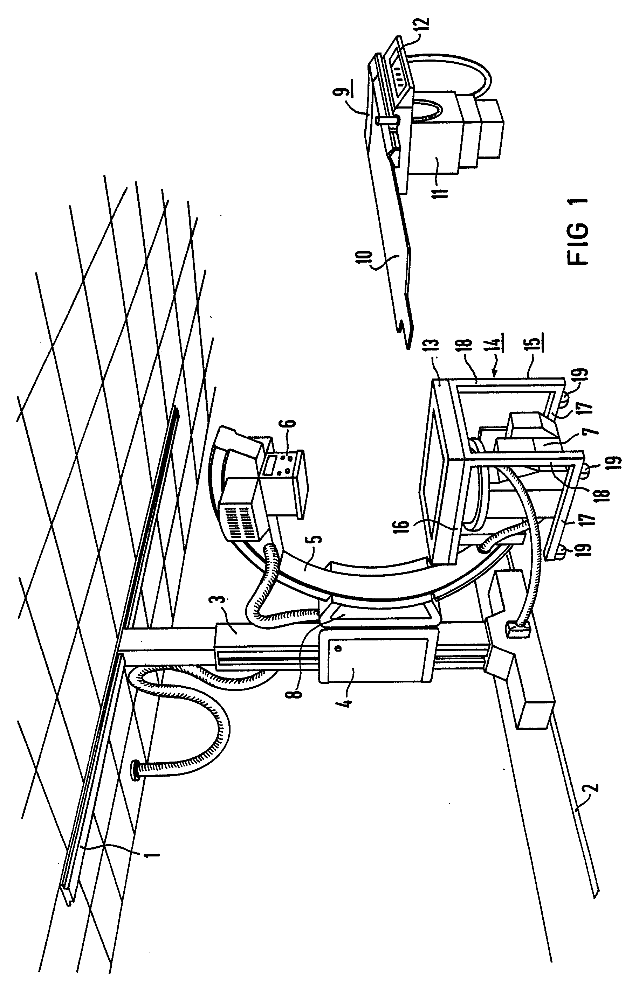

In der Fig. 1 ist eine erfindungsgemäße Röntgendiagnostikanlage dargestellt. Diese weist eine in einer Decken- und einer Bodenschiene 1 und 2 verfahrbare vertikale Säule 3 auf, an der ein Wagen 4 höhenverstellbar angebracht ist. An diesem ist ein C-Bogen 5 angebracht, der einen Röntgenstrahler 6 und einen Röntgenbildverstärker 7 an seinen einander gegenüberliegenden Enden trägt. Der C-Bogen 5 ist in einem Halter 8 längs seines Umfanges verstellbar, wobei der Halter 8 und damit der C-Bogen 5 um eine horizontale Achse schwenkbar mit dem Wagen 4 verbunden ist. Außerdem ist eine Patientenliege 9 vorgesehen, die eine Patientenlagerungsplatte 10 aufweist, die unter anderem höhenverstellbar auf einem Sockel 11 angebracht ist. Die Decken- und die Bodenschiene 1 und 2 verlaufen parallel zu der Längsachse der Patientenlagerungsplatte 10, so daß der Röntgenstrahler 6 und der Röntgenbildverstärker 7 gemeinsam längs der Patientenlagerungsplatte 10 verstellbar sind.

An der Patientenliege 9 ist ein Bedienpult 12 angebracht, das Bestandteil einer nicht dargestellten Steuerungseinrichtung ist, mittels derer die Bewegungen der Säule 3, des Wagens 4, des Halters 8, des C-Bogens 5 und der Patientenlagerungsplatte 10, die in nicht dargestellter Weise motorisch bewirkt werden, steuerbar sind.

Die erfindungsgemäße Röntgendiagnostikanlage besitzt außerdem einen Blattfilmwechsler 13, so daß ein auf der Patientenlagerungsplatte 10 befindliches Untersuchungsobjekt wahlweise mittels des Röntgenbildverstärkers 7 und einer diesem nachgeschalteten, nicht dargestellten Röntgenfernsehkette durchleuchtet oder mittels des Blattfilmwechslers 13 aufgenommen werden kann.

Dieser besitzt ein Fahrgestell 14 mit einem U-förmigen Rahmen 15, der zwei obere horizontale Schenkel 16, zwei untere horizontale Schenkel 17 und zwei diese jeweils verbindende vertikale Schenkel 18 aufweist. Der Blattfilmwechsler 13 ist an den oberen horizontalen Schenkeln 16 angebracht, während die unteren horizontalen Schenkel 17 mit Rädern 19 versehen sind.

Zur Anfertigung von Aufnahmen wird der Blattfilmwechsler 13 in der in Fig. 1 dargestellten Weise mittels des Fahrgestells 14 im Strahlengang vor dem Röntgenbildverstärker 7 positioniert, wobei der Rahmen 15 des Fahrgestells den Röntgenbildverstärker 7 umgreift. Dabei ist der Blattfilmwechsler 13 mit dem Röntgenbildverstärker 7 in in Fig. 1 nicht sichtbarer Weise derart kuppelbar, daß er Bewegungen des Röntgenbildverstärkers 7 längs der Patientenlagerungsplatte 10 auf seinem Fahrgestell 14 folgt. Die dazu vorgesehenen Kupplungsmittel sind derart ausgebildet, daß der Blattfilmwechsler 13 zu dem Röntgenstrahler 6 mittig ausgerichtet ist und den Bewegungen des Röntgenbildverstärkers 7 längs der Patientenlagerungsplatte 10 starr folgt. Dazu ist es erforderlich, daß die Kupplungsmittel die auftretenden Beschleunigungskräfte übertragen können.

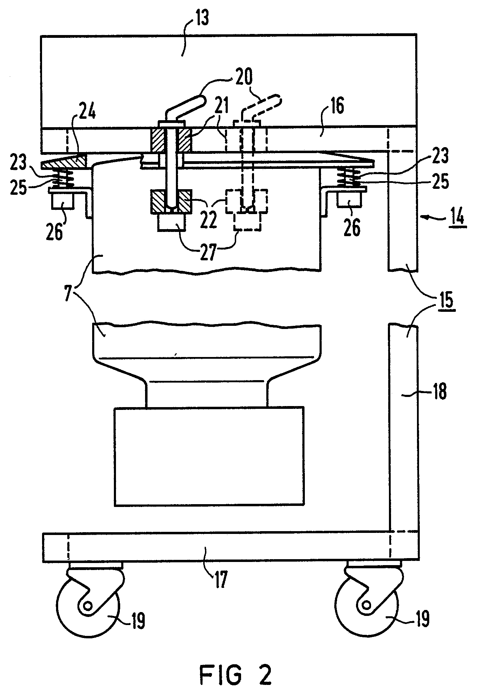

Solange die Röntgendiagnostikanlage im Durchleuchtungsbetrieb betrieben wird, ist der Blattfilmwechsler 13 abgekuppelt und mittels seines Fahrgestells 14 in eine Parkposition verfahren, in der er das Bedienungspersonal nicht behindert. Sobald eine Aufnahme angefertigt werden soll, wird der Blattfilmwechsler 13 an die Röntgendiagnostikanlage herangefahren und mit dem Röntgenbildverstärker 7 gekuppelt. Um diesen Vorgang zu erleichtern besteht die Möglichkeit, den Röntgenbildverstärker 7 in eine spezielle Kuppelposition zum Ankuppeln des Blattfilmwechslers 13 zu verfahren. Wie aus Fig. 2 ersichtlich ist, befindet sich der Röntgenbildverstärker 7 dann in einer solchen Höhe, daß der Blattfilmwechsler 13 mittels seines Fahrgestells 14 gerade noch über den Röntgenbildverstärker 7 gefahren werden kann. Der Röntgenbildverstärker 7 und der Blattfilmwechsler 13 können dann miteinander gekuppelt werden, indem zwei Kupplungsbolzen 20, die in an den oberen horizontalen Schenkeln 16 des Rahmens 15 angebrachten Führungen 21 längsverschiebbar sind, in die Bohrungen zweier an dem Röntgenbildverstärker 7 angebrachter Augen 22 eingeführt werden.

Wie aus Fig. 2 weiter ersichtlich ist, ist an dem dem Blattfilmwechsler 13 zugewandten Ende des Röntgenbildverstärkers 7 ein auf Federn 23 nachgiebig gelagerter Ring 24 vorgesehen, der mit den horizontalen Schenkeln 16 des Rahmens 15 in Eingriff kommt und durch diese gegen die Wirkung der Federn um ein gewisses Maß in Richtung auf den Röntgenbildverstärker 7 niedergedrückt wird, sobald damit begonnen wird, den Blattfilmwechsler 13 vor dem Röntgenbildverstärker 7 zu positionieren. An dem Ring 24 sind Stößel 25 angebracht, die mehrere über den Umfang des Röntgenbildverstärkers 7 verteilte Schalter 26 betätigen, sobald der Ring 24 niedergedrückt wird. Außerdem sind zwei Schalter 27 vorgesehen, die jeweils durch einen der Kupplungsbolzen 20 betätigt werden, sobald dieser vollständig in die Bohrung des entsprechenden Auges 22 eingeführt ist.

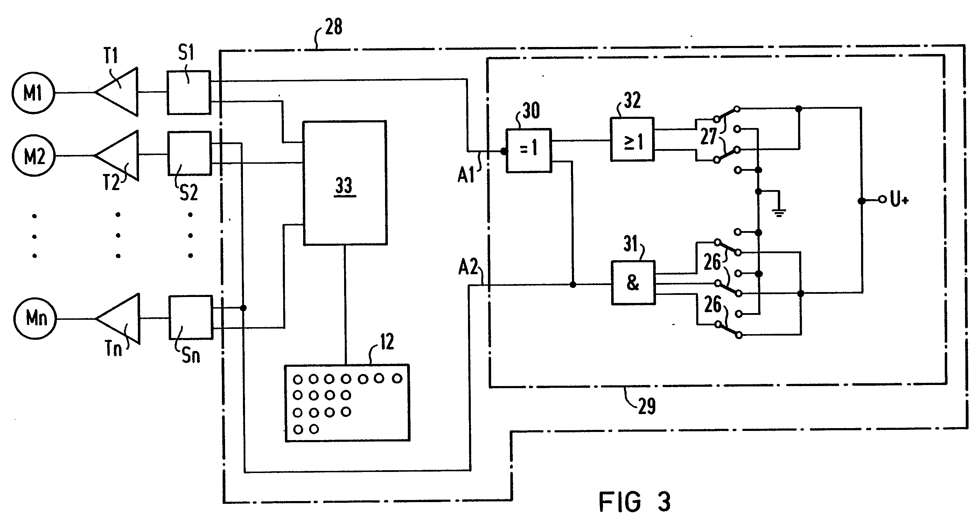

Der Ring 24, die Federn 23, die Stößel 25 sowie die Schalter 26 und 27 sind Teil der Steuerungseinrichtung, die in Fig. 3 schematisch veranschaulicht ist.

In dieser Fig. sind Motore M1, M2 bis Mn dargestellt, die die obengenannten Gerätebewegungen bewirken. So ist z.B. der Motor M1 dazu bestimmt, die Säule 3 längs der Patientenliege 9 zu verfahren. Jedem der Motore M1 bis Mn ist ein Treiber T1 bis Tn zugeordnet. Die Treiber T1 bis Tn stehen über Schaltstufen S1 bis Sn, z.B. Relais, mit der Steuerungseinrichtung 28 in Verbindung, die eine Steuereinheit 33 und das an diese angeschlossene Bedienpult 12 umfaßt, so daß durch geeignetes Betätigen der Tasten des Bedienpultes 12 die einzelnen Gerätebewegungen in der einen oder anderen Richtung hervorgerufen werden können.

Wie Fig. 3 zeigt, umfaßt die Steuerungseinrichtung 28 außerdem eine insgesamt mit 29 bezeichnete Sicherheitseinrichtung, die zwei Ausgänge A1 und A2 besitzt, von denen der Ausgang A1 mit der Schaltstufe S1 und der Ausgang A2 mit den Schaltstufen S2 bis Sn verbunden ist. Bestandteil der Sicherheitseinrichtung 29 sind außer den im Zusammenhang mit der Fig. 2 beschriebenen mechanischen Bauteilen, den Schaltern 26, von denen in Fig. 3 drei dargestellt sind, und den beiden Schaltern 27 eine EXNOR-Schaltung 30, eine UND-Schaltung 31 und eine ODER-Schaltung 32. Die Schalter 26 sind mit den Eingängen der UND-Schaltung 31 verbunden, während die Schalter 27 mit den Eingängen der ODER-Schaltung 32 verbunden sind. Die Schalter 26 und 27 sind jeweils als Umschalter ausgeführt und derart geschaltet, daß die Eingänge der UND-Schaltung 31 und der ODER-Schaltung 32 je nach Schaltstellung des zugehörigen Schalters 26 oder 27 wahlweise auf ein positives Potential U+ (high) oder Erdpotential (low) gelegt werden können. Der Ausgang A2 der Sicherheitseinrichtung 29 ist unmittelbar durch den außerdem mit dem einen Eingang der EXNOR-Schaltung 30 verbundenen Ausgang der UND-Schaltung 31 gebildet. Der Ausgang A1 der Schutzeinrichtung 29 entspricht dem Ausgang der EXNOR-Schaltung 30, deren anderer Eingang mit dem Ausgang der ODER-Schaltung 32 verbunden ist.

Bezüglich der Schalter 26 und 27 ist die Anordnung derart getroffen, daß dann, wenn sich der Blattfilmwechsler 13 nicht vor dem Röntgenbildverstärker 7 befindet und demzufolge auch nicht an diesen gekuppelt ist, alle Schalter 26 und 27 eine solche Schaltstellung einnehmen, daß die Eingänge der UND-Schaltung 31 und der ODER-Schaltung 32 und somit auch deren Ausgänge high sind. Dies hat zur Folge, daß der Ausgang A2 der Sicherheitseinrichtung 29 sowie die beiden Eingänge der EXNOR-Schaltung 30 und damit auch der Ausgang A1 high sind. Sämtliche Schaltstufen S1 bis Sn befinden sich nun in einem Zustand, in dem sie Steuersignale der Steuerungseinrichtung 28 zu den Treibern T1 bis Tn durchlassen. Mittels des Bedienpultes 12 und der Steuereinheit 33 können somit beliebige Gerätebewegungen motorisch bewirkt werden.

Sobald der Blattfilmwechsler 13 vor dem Röntgenbildverstärker 7 positioniert wird, wird wenigstens einer der Schalter 26 über den Ring 24 und den entsprechenden Stößel 25 betätigt, so daß der entsprechende Eingang der UND-Schaltung 31 auf low liegt, was zur Folge hat, daß deren Ausgang und mit diesem der Ausgang A2 der Sicherheitseinrichtung 29 ebenfalls auf low geht. Dies gilt auch für den Ausgang A1 der Sicherheitseinrichtung 29, da an den Eingängen der EXNOR-Schaltung 30 nun unterschiedliche Pegel liegen. Die Schaltstufen S1 bis Sn nehmen infolge des Umstandes, daß beide Ausgänge A1 und A2 der Sicherheitseinrichtung 29 auf low liegen, einen Schaltzustand ein, in dem die Weiterleitung von Steuersignalen von der Steuerungseinrichtung 28 an die Treiber T1 bis Tn unterbunden ist, so daß jegliche motorische Gerätebewegung blockiert ist.

Wird der vor dem Röntgenbildverstärker 7 positionierte Blattfilmwechsler 13 mit diesem mittels der Kupplungsbolzen 20 gekuppelt, werden auch die Schalter 27 betätigt, was zur Folge hat, daß auch der Ausgang der ODER-Schaltung 32 auf low geht, so daß nun beide Eingänge der EXNOR-Schaltung 30 auf low liegen, was zur Folge hat, daß der Ausgang A1 der Sicherheitseinrichtung 29 wieder auf high geht. Dies wiederum bewirkt, daß die Schaltstufe S1 in einen Schaltzustand versetzt wird, in dem Steuersignale von der Steuerungseinrichtung 28 zu dem Treiber T1 gelangen können, die wie oben erwähnt mit Hilfe des Motors M1 eine Verschiebung der Säule 3 und damit den Röntgenstrahlers 6 und des Bildverstärkers 7 sowie des mit diesem gekuppelten Blattfilmwechslers 13 längs der Patientenliege bewirken. Da der Ausgang A2 der Sicherheitseinrichtung 29 weiterhin auf low liegt, sind alle weiteren Gerätebewegungen nach wie vor blockiert. Damit der Ausgang der ODER-Schaltung 32 auf low geht, ist es erforderlich, daß deren beide Eingänge auf low liegen. Dies hat zur Folge, daß die motorische Verschiebung der Säule 3 längs der Patientenliege 9 nur dann möglich ist, wenn der Blattfilmwechsler 13 mittels beider Kupplungsbolzen 20 korrekt mit dem Röntgenbildverstärker 7 gekuppelt ist.

Sowie der Blattfilmwechsler 13 von dem Röntgenbildverstärker 7 wieder abgekuppelt wird, sind wieder sämtliche Gerätebewegungen blockiert. Wird der Blattfilmwechsler 13 von dem Röntgenbildverstärker 7 wieder entfernt, gehen die Ausgänge A1 und A2 der Sicherheitseinrichtung 29 wieder auf high, so daß sämtliche Gerätebewegungen uneingeschränkt möglich sind.

Die im Zusammenhang mit dem Ausführungsbeispiel beschriebene Ausbildung der Kupplungsmittel, der Steuerungseinrichtung, der Sicherheitseinrichtung und des Fahrgestells des Blattfilmwechslers ist jeweils nur beispielhaft zu verstehen.

## Ansprüche

1. Röntgendiagnostikanlage zur wahlweisen Durchleuchtung oder Aufnahme eines Untersuchungsobjektes mit einem Röntgenbildverstärker (7) mit nachgeschalteter Fernsehkette, einem Blattfilmwechsler (13) sowie einem Röntgenstrahler (6) und einer Liege (9) für das Untersuchungsobjekt, wobei der Blattfilmwechsler (13) ein Fahrgestell (14) aufweist, **dadurch gekennzeichnet,** daß der Röntgenstrahler (6) und der Röntgenbildverstärker (7) gemeinsam längs der Liege (9) verschiebbar sind und daß der Blattfilmwechsler (13) mittels des Fahrgestells (14) im Strahlengang vor dem Röntgenbildverstärker (7) positionierbar und mit diesem derart kuppelbar ist, daß er Bewegungen des Röntgenbildverstärkers (7) längs der Liege (9) auf seinem Fahrgestell (14) folgt.

2. Röntgendiagnostikanlage nach Anspruch 1, bei der der Röntgenbildverstärker (7) und der Röntgenstrahler (6) an entgegengesetzten Enden eines allseitig schwenkbaren und höhenverstellbaren bogenförmigen Trägers (5) angebracht und Mittel (M1, M2 bis Mn) zum motorischen Bewegen des Trägers (5) und eine Steuerungseinrichtung (28) für die Bewegungen des Trägers (5) vorgesehen sind, **dadurch gekennzeichnet,** daß der Röntgenbildverstärker (7) in eine Kuppelposition zum Ankuppeln des Blattfilmwechslers (13) verfahrbar und die Steuerungseinrichtung (28) derart ausgebildet ist, daß in der Kuppelposition bei vor dem Röntgenbildverstärker (7) befindlichem Blattfilmwechsler (13) sämtliche Bewegungen des Trägers (5) bis zum erfolgten Ankuppeln des Blattfilmwechslers (13) blockiert und nach erfolgtem Ankuppeln des Blattfilmwechslers (13) Bewegungen des Trägers (5) nur längs der Liege (9) möglich sind.

3. Röntgendiagnostikanlage nach Anspruch 2, **dadurch gekennzeichnet,** daß mit der Steuerungseinrichtung (28) zusammenwirkende erste Mittel (24, 25, 26), mittels derer die Anwesenheit des Blattfilmwechslers (13) vor dem Röntgenbildverstärker (7) feststellbar ist, und zweite Mittel (20, 27) vorgesehen sind, mit deren Hilfe das erfolgte Ankuppeln des Blattfilmwechslers (13) an den Röntgenbildverstärker (7) feststellbar ist.

4. Röntgendiagnostikanlage nach Anspruch 2 oder 3. **dadurch gekennzeichnet,** daß sich der Röntgenbildverstärker (7) in Kuppelposition unterhalb der Liege (9) befindet.

5. Röntgendiagnostikanlage nach einem der Ansprüche 1 bis 4, **dadurch gekennzeichnet,** daß das Fahrgestell (14) einen den Röntgenbildverstärker (7) in Kuppelposition umgreifenden U-förmigen Rahmen (15) mit zwei horizontalen und einem diese verbindenden vertikalen Schenkel (16, 17 bzw. 18) aufweist, wobei der eine horizontale Schenkel (16) den Blattfilmwechsler (13) trägt und der andere horizontale Schenkel (17) mit Rädern (19) versehen ist.

FIG 1

EP 0 297 355 A1

FIG 2

FIG 3

EP 0 297 355 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | SIEMENS DRUCKSCHRIFT "SIREGRAPH C", Oktober 1982, Seiten 1-12, Best. Nr. M-R 18/7262-02, Erlange, DE<br>* Seiten 1-8 *<br>--- | 1 | A 61 B 6/00 |
| A | --- | 4 | |
| Y | DE-A-2 237 272 (SIEMENS)<br>* Seite 1, Zeilen 1-4; Seite 4, Zeile 13 - Seite 5, Zeile 11; Seite 6, Zeilen 16-21; Figur 1 *<br>--- | 1 | |
| A | WO-A-8 606 267 (H.G. ENDER)<br>* Zusammenfassung; Seite 5, Zeile 24 - Seite 6, Zeile 8; Figuren 1,2 *<br>--- | 1 | |
| A | DE-A-1 939 557 (VEB TRANSFORMATOREN- UND RÖNTGENWERK DRESDEN)<br>* Seite 1, Zeilen 1-5; Seite 6, Zeilen 5-19; Seite 6, Zeile 25 - Seite 7, Zeile 12; Figuren *<br>--- | 2 | |
| A | DE-A-1 416 863 (KOCH UND STERZEL)<br>* Seite 3, Zeilen 3-17; Figuren *<br>--- | 2 | RECHERCHIERTE SACHGEBIETE (Int. Cl.4)<br><br>A 61 B<br>G 03 B |
| A | FR-A-2 110 008 (SIEMENS)<br>* Seite 1, Zeilen 1-10; Seite 3, Zeilen 7-15; Seite 4, Zeilen 1-20; Figuren 1,2 *<br>----- | 3 | |

Der vorliegende Recherchenbericht wurde für alle Patentansprüche erstellt

| Recherchenort | Abschlußdatum der Recherche | Prüfer |
|---|---|---|
| DEN HAAG | 04-10-1988 | FERRIGNO, A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
........................................................
& : Mitglied der gleichen Patentfamilie, übereinstimmendes Dokument

EPO FORM 1503 03 82 (P0403)